(19) Europäisches Patentamt
European Patent Office
Office européen des brevets

(11) Publication number: 0 198 765 A2

# (12) EUROPEAN PATENT APPLICATION

(21) Application number: 86400723.2

(22) Date of filing: 03.04.86

(51) Int. Cl.[4]: A 61 K 9/50

(30) Priority: 09.04.85 US 721410

(43) Date of publication of application: 22.10.86 Bulletin 86/43

(84) Designated Contracting States: AT BE CH DE FR GB IT LI LU NL SE

(71) Applicant: GEORGETOWN UNIVERSITY, 37th and "O" Streets, N.W., Washington, D.C. 20057 (US)

(72) Inventor: Rahman, Aquilur, 6504 Farmingdale Court, Rockville MD 20855 (US)

(74) Representative: Mongrédien, André et al, c/o SOCIETE DE PROTECTION DES INVENTIONS 25, rue de Ponthieu, F-75008 Paris (FR)

## (54) Preparation of liposomes.

(57) A process for encapsulating a drug or physiologically active substance in a liposome, which comprises forming a dispersion of a liposome-forming lipid, a physiologically active substance, and a lipophilic chelating agent, wherein liposomes are produced in the dispersion, and recovering the liposome-encapsulated drug or physiologically active substance from the dispersion, is disclosed along with liposomes produced in this manner.

EP 0 198 765 A2

# Description

## Preparation Of Liposomes

### Technical Field

The present invention relates to the preparation of liposome-encapsulated materials and in particular to the encapsulation of drugs and other physiologically active substances in liposomes.

### Background Art

As is widely recognized, many drugs and other physiologically active substances have unfortunate side-effects which can cause pain, discomfort, injury or even death to the recipient. This is particularly true with drugs designed to treat tumors and various types of cancers. Such drugs, in order to be effective, must be very potent, but the potency which makes them effective as an anti-cancer or anti-tumor agent also presents a significant danger to normal tissue in the body. For example, adriamycin, a widely used anthracycline glycoside, is known to be useful in treating leukemias and solid tumors. This drug also attacks the heart muscle, however, causing irreversible injury. As a result both the dose rate and total dosage of this drug must be carefully controlled and limited so as to avoid excessive injury to the heart muscle.

Several workers have suggested encapsulating adriamycin and its related anthracycline glycosides in liposomes to reduce cardiotoxicity. One such technique is described in United States Patent 3,993,754 and is typical of those which have been suggested in the art. Typical yields with such processes, even with effectively encapsulated materials, are on the order of about 5%. This means that 95% percent or more of the active drug remains unencapsulated and must be separated from the encapsulated material and recycled if possible. Quite often a portion of the active drug is lost forever during purification and recycling. As a result of these low encapsulation efficiencies, liposome encapsulation of drugs to reduce toxicity has not been considered practical on a commercial scale.

The existence of stearylamine covalently coupled to diethylenetriaminepentaacetic acid, a compound that can be used in the practice of the present invention, was known prior to the present invention. Hnatowich et al. J. Nucl. Med., 22, 810-814 (1981), discloses the synthesis of this compound and the use of the stearylamine-DTPA compound to label liposomes by complexation with radioactive metal ions. These liposomes were used in the typical fashion as radioactive tracers and were not used to encapsulate drugs or any other materials that were active pharmacologically.

United States Patent 4,419,348 discloses novel encapsulated anthracycline glycosides and a process for their preparation. Utilizing the technique of United States Patent 4,419,348, it has been found possible to obtain yields of encapsulated anthracycline glycosides on the order of from 25 to 55 percent. This marked increase in effective yield has prompted commercial interest in the encapsulated liposomes disclosed in U.S. Patent 4,419,348.

Unfortunately, the technique of this patent appears to be limited to use with anthracycline glycosides and does not appear to be satisfactory for use with other types of drugs. For example, it would be highly desirable to be able to encapsulate mitoxantrone, m-AMSA, actinomycin, mitomycin-C and cis-platinum to reduce the toxicity of these drugs. However, liposome encapsulation with these drugs using previously known techniques generally resulted in a yield of only about 5%. Furthermore, it would also be desirable to improve the yield of the encapsulated anthracycline glycoside-cardiolipin complex described in United States Patent 4,419,348.

Accordingly, a need continues to exist for a technique of encapsulating drugs and other physiologically active substances in liposomes at high yields.

## Disclosure of the Invention

Accordingly, it is an object of the present invention to provide a process for encapsulating drugs and other physiologically active materials in liposomes at high yields.

Within this broad objective, it is also an object of the present invention to provide a technique for encapsulating lipid-soluble drugs and physiologically active substances at high yields in liposomes.

It is yet another object of the present invention to provide a technique for encapsulating drugs and physiologically active substances which are both water and lipid soluble in liposomes at higher yields than is presently possible.

Yet another object of the present invention is to provide a technique for encapsulating water-soluble drugs and physiologically active substances in liposomes at high yields.

These and other objects of the present invention which will become obvious from the description which follows have been achieved by a process which comprises incorporating into a liposome a lipophilic chelating agent comprising a portion which is lipophilic and capable of inserting into a liposome bilayer and a portion capable of acting as a chelating agent. The physiologically active substance together with the constituents of the liposome and the lipophilic chelating agent are subjected to conventional liposome-forming techniques to produce the encapsulated drug. It has been found that encapsulation yields are increased substantially through the use of this technique involving a liphophilic chelating agent.

## Brief Description of the Drawings

FIGURE 1 is a graph showing plasma pharmacokinetic levels of free and liposome-entrapped daunorubicin in mice.

FIGURE 2 is a graph showing daunorubicin disposition in mice hearts following intraveneous administration of free and liposome-entrapped drug.

FIGURE 3 is a graph showing plasma pharmacokinetic levels of free and liposome-entrapped doxorubicin in mice.

FIGURE 4 is a graph showing doxorubicin disposition in mice hearts following intraveneous administration of free and liposome-entrapped drug.

FIGURE 5 is a graph showing comparative toxicity of free and liposome-entrapped doxorubicin in mice.

Best Mode for Carrying Out the Invention

In the present invention, substantially any lipophilic chelating agent may be used which does not have excessive adverse toxicity effects on the host organism to which the encapsulated material is to be administered. Some toxicity can be tolerated as long as the lipophilic chelating agent will have no adverse toxilogical effects at the amounts of chelating agent which are being employed in the present invention.

A chelating agent is typically a compound containing donor atoms that can combine by coordinate bonding with an acceptor atom, typically a metal atom or ion, to form a cyclic structure called a chelation complex or, more simply, a chelate. When coordinate bond formation occurs between the acceptor and donor atoms, the atoms of the ligand that connect the donor atoms complete a ring that gives the structure its chelate character. Each additional donor atom of the ligand that coordinates with the electron acceptor forms another ring. A chelating agent can be bidentate, tridentate, tetradentate, and so on, depending on whether it containd 2, 3, 4, or more donor atoms capable of complexing with the acceptor atom. The principal donor atoms are nitrogen, oxygen, and sulfur, but phosphorous, arsenic, and selinium also are capable of forming chelates. Chelates can also be identified by the number of donor atoms which surround the central acceptor atom. The most common coordination numbers are 4 and 6, with 2 and 8 being less common.

Typical chelating agents known in the art prior to the present invention are water-soluble compounds lacking the lipophilic portion that is essential to the practice of the present invention. In their typical prior art uses, chelating agents were used to make water-soluble ccoordination complexes of metal ions and accordingly were required to be water-soluble. Although the lipophilic chelating agents of the present invention retain a chelating portion essentially identical to the chelating portion of a water-soluble compound, they will also contain a lipophilic portion capable of inserting into a liposome lipid bilayer. The lipophilic chelating layer will typically have a formula R-K in which R is a lipophilic portion and K represents the remainder of the molecule comprising the chelating portion.

Preferred chelating groups for forming the chelating portion of R-K are those containing carboxylic acid groups, such as amino-carboxylic acids, hydroxycarboxylic acids, and similar compounds. However, many other chelating agents are known and are disclosed in numerous references, such as the Kirk-Othmer Encyclopedia of Chemical Technology, Third Edition, Volume 5, John Wiley & Sons, New York, pages 339-368 (which is herein incorporated by reference) and the publications disclosed therein. Any of known types of chelating agents can be used to form the chelating portion of R-K as long as undue toxicity (readily determined by simple experimentation) does not result from use of that particular type of chelating portion. Examples of amino carboxylic acids are ethylenediaminetetraacetic acid, hydroxyethylethylenediaminetriacetic acid, nitrilotriacetic acid, N-dihydroxyethylglycine, and ethylenebis(hydroxyphenylglycine). Examples of hydroxycarboxylic acids are tartaric acid, citric acid, gluconic acid, and 5-sulfosalicylic acid.

Particularly preferred are polycarboxylic acid chelating agents, particularly the acetate derivatives of ethylenediamine and related compounds. Examples of such chelating agents include diethylenetriaminepentaacetic acid (DTPA), ethylenediaminetetracetic acid (EDTA) ethylenediamine-N,N'-diacetic acid (EDDA), ethylenediaminedi(acetic acid)di(propionic acid) (EDDADP), ethyleneglycoldiacetic acid (EGTA), and similar related compounds. In general, preferred polycarboxylic acid chelating portions of the invention have a molecular weight of at least about 200 and preferably at least about 250 daltons. Preferred polycarboxylic acid chelating agents contain at least 3 carboxylic acid groups, preferably at least 4 carboxylic acid groups, and more preferably 5 or more carboxylic acid groups.

The lipophilic portion of the chelating agent can be derived from any lipophilic compound having suitable toxilogical properties. Lipophilic organic groups capable of forming the R portion of the lipophilic chelating agent R-K will readily be recognized by those of orginary skill in the field of organic chemistry. Lipophilicity can readily be estimated by assuming that the portion K is replaced by a hydrogen to give a hypothetical compound of formula RH. If the compound RH is more soluble in an organic solvent (especially benzene, chloroform, acetone, ether, or ethylacetate) than it is in water, it is considered to be lipophilic, and the R-portion of a compound R-K will likewise be lipophilic.

However, it will be recognized that many small hydrocarbons, although lipophilic in themselves, are not sufficiently large to anchor a molecule of the formula R-K in a liposome bilayer when K represents a relatively large chelating portion of a molecule, the chelating portion typically being highly hydrophilic. Accordingly, it is preferred that R comprises a hydrocarbyl portion comprising at least six and more preferably at least 12 carbon atoms. It is preferred that R not represent an unsubstituted aromatic hydrocarbon because of possible dangers of toxicity. However, it will be readily recognized that other types of hydrocarbyl groups, such as steroids, cycloalkanes and the like can readily be used. For example, the hydroxyl group of chloresterol can easily be attached to a chelating molecule. Fatty acid alcohols and fatty acid amines, containing fatty acid alkyl portions that are preferred for use in the practice of this invention, can also readily be coupled to a chelating agent through a condensation reaction when the chelating agent contains a carboxylic acid group. Compounds of the formula R-K formed in this manner represent a preferred embodiment of the invention.

This preferred aspect of invention is practiced by coupling a lipophilic compound containing a function group capable of reacting with a carboxylic acid group with a chelating compound containing a carboxylic acid group. Typical lipophilic compounds used in this aspect of the invention include alcohols and primary and secondary amines. For the purposes of this application, a compound of formula ROH, $RNH_2$, or $R_2NH$ is lipophilic if it is more soluble in an organic solvent (especially benzene, chloroform, acetone, ether, or ethylacetate) than it is in water. Many lipophilic compounds and their solubilities are already known. However, the lipophilicity of any compound whose lipid character is not already known can readily be determined by measuring solubility in water and an organic solvent. It is preferred that such compounds are highly lipophilic so as to enhance the solubility of the resulting condensation (or other type of coupling) product in the lipid bilayer phase. A compound is highly lipophilic if its solubility in any of the organic solvents listed above is at least three times that of its solubility in water. If a practitioner wishes to carry out this aspect of the invention with a lipophilic compound not specifically described herein, he may readily determine whether a bond is formed with the chelating compound by mixing or otherwise reacting the water-soluble chelating agent and lipophilic compound in a manner described below and following formation of a bond by infrared spectrophotometry. When a preferred chelating agent having a carboxylic acid chelating group is used, formation of a complex can readily be detected by a shift in the absorbance position of the carbonyl group.

Preferred lipophilic compounds of the invention for use in preparing lipophilic chelating agents are fatty acid amines. Many fatty acid amines are known and others can readily be produced by forming the amide of a fatty acid and reducing the amide to an amine.

Primary amines are preferred, but secondary amine are also suitable. If a secondary amine is utilized, it is preferred that one large alkyl chain be present and that the remaining nitrogen substituent be small, preferably a methyl or ethyl group. Preferred fatty amines are those which exist in a solid state when pure at a temperature of 25°C. Particularly preferred fatty amines include dodecylamine, tetradecylamine, hexadecyl amine, octadecylamine, icosylamine, and docosylamine. Unsaturated fatty acid amines (e.g., those corresponding to oleic and linoleic) are also usable in the practice of this invention.

As indicated by these particularly preferred compounds, preferred lipophilic compounds are those having a single functional group capable of reacting with the water-solable chelating compound to form a bond and an alkyl or alkenyl portion. However, other functional groups may be present provided that the resulting lipophilic compound is not toxic to the host organism being treated at the dosage at which the lipophilic chelating agent is administered. Naturally, other functional groups present in the lipophilic molecule must not be so extensive or of a type that destroys the lipophilic nature of the lipophilic chelating agent as described above.

In preparing the lipophilic agent, the ratio or reactants is not critical; however, when bonding is accomplished using the reactivity of functional groups in the respective compound to accomplish bonding, it is preferred to use an excess of the less expensive component so as to form a bond with all of the more expensive component (usually the chelating agent). The specific ratios will depend upon how many moles of lipophilic compound are to be bound with each mole of chelating agent. For example, when one mole of water-soluble chelating agent binds with one mole of lipophilic compound, approximately equal molar amounts of each are reacted, with preferably a slight excess of lipophilic compound typically being employed. This is the typical situation and accordingly represents a preferred embodiment of the invention.

Although many preferred embodiments of the present invention can be prepared by a simple condensation reaction between, for example, a carboxylic acid group in a chelating agent, and a reactive group, such as an amino or hydroxy group, in a lipophilic compound, it is recognized that other chelating agents covalently coupled to long-chain alkyl or alkenyl groups in other manners are equivalent to the compounds disclosed above as long as chelating and lipophilic properties are both retained. Many such compounds and methods of producing them can readily be envisioned. For example, stearyl alcohol, itself readily available from stearic acid, can be converted to stearylchloride using known techniques. Stearylchloride can be reacted with ethylenediamine using conventional techniques to produce (2-aminoethyl)-N-octadecylamine. This and similar compounds can be used in the production of polycarboxylic acid amines in a manner similar to that used to produce EDTA and DTPA. Because of the ease with which reactions with a carboxylic acid group (typically in the form of an anhydride) in a polycarboxylic acid chelating agent can take place, it will be recognized that reactions formed in this manner are preferred. Any functional group that will react with a carboxylic acid group (such as an alcohol or amine) can be reacted with a chelating agent (optionally as an acid anhydride or chloride) containing an excess of carboxylic acid groups over and beyond that necessary for chelating in order to produce compounds that can be used in the practice of the present invention. For example, a 1:1 molar ratio of stearyl amine and DTPA anhydride forms one amide bond on reaction, leaving the remaining four carboxylate groups available for functioning as chelating groups.

While it is not known at the present time precisely how the lipophilic chelating agent functions in combination with the lipophilic compound to increase the yield of the encapsulation process, it is speculated that the chelating agent may serve one or more of the following functions. When the drug to be encapsulated is lipid soluble, more drug is introduced into the lipid bilayer because the lipophilic chelating agent is capable of stabilizing the lipid bilayer, thereby counteracting the instability which may be introduced by the presence of a soluble drug in the bilayer. When the drug is water soluble and capable of being chelated, the chelating portion of the molecule can chelate with the drug, thereby causing some of the drug to become soluble in a lipid bilayer and increasing the amount of drug that can be retained in the liposome. Some of the drug may become encapsulated in the water portion in the interior of the liposome, while additional drug is solubilized in the lipid bilayer. This is particularly important when the drug is only water soluble, since the amount of water which is contained in a liposome is generally very small when compared with the amount of lipid material involved. When the drug is both lipid and water soluble the presence of a chelating agent serves to increase the amount of drug which can be retained in the lipid bilayer by stabilizing the material.

In selecting specific chelating agents for specific drugs, several factors can be considered. When the drug is either lipid soluble or both water and lipid soluble, the choice of a lipephilic chelating agent is limited only by its ability to insert into the liposome's lipid bilayer. That is, substantially any lipophilic chelating agent may be used since where the drug is either lipid soluble or both lipid and water soluble, the objective of the chelate complex is primarily to stabilize the bilayer. On the other hand, if the drug is only water soluble then it is desirable that the chelating agent not only be capable of inserting into the lipid bilayer but also of interacting with the drug to form a complex. Drugs which are chelated by the lipophilic chelating agent may be considered to be "drawn" into the bilayer, thereby increasing the amount of water soluble drug which may be held by the liposome. Cytotoxic metals and metal complexes, which are of increasing use in cancer chemotherapy, are examples of water-soluble drugs capable of undergoing complex formation with the chelating group. An example of a water-soluble drug of this type is cis-platinum. Thus, when cis-platinum is to be used, a chelating agent such a DTPA coupled to a lipophilic compound should be used since DTPA can bond with the lipophilic compound and still retain sufficient functionality to form a complex with cis-platinum.

On the other hand, with drugs such as actinomycin-D and mitomycin-C which are mostly lipid soluble, an interaction directly between the chelating agent and the drug is not necessary and substantially any chelating agent can be used. With these drugs it is primarily the stabilization of the lipid bilayer which is important rather than complexing with the drug itself. When utilizing anthracycline glycoside, which is both water and lipid soluble, one can use either a chelating agent which does interact with the drug or one which does not. With such compounds there is a slight advantage to utilizing a chelating agent which will interact with the drug since that may increase the amount of drug which will be encapsulated.

It is not necessary to be able to predict from the structure of the drugs involved whether complexation with a lipophilic chelating agent will occur. This can be determined readily by emperical testing. The present invention does not involve any difficult steps in complex formation between the drug and chelating agent, since all complexes described herein are readily produced by mixing the components with each other, as is typical of chelation reacting. If any bonding reaction takes place, that reaction takes place as a result of a mixing without further intervention. Accordingly, mere mixing of the chelating agent and the drug followed by simple detection of the formation of a complex readily enables an investigator to determine whether a complex of the drug with the chelating agent has occurred. Complex formation can readily be detected using spectroscopic means.

The therapeutic efficacy of the material which is encapsulated is substantially the same as, or is better than, that of the free, unencapsulated material. Accordingly, the amount of a pharmacologically active material that is to be formed into liposomes for administration to a patient can readily be determined from the amount of the material that would be normally administered without formation of liposomes.

The materials which will form the actual liposome bilayer will be selected based upon the intended use of the drug. It has often been found that by selecting suitable constituents for the liposomes, it is possible to prepare materials which are site selective. A discussion of liposomes, their formation and usage is found, for example, in the Annals of the New York Academy of Sciences, Volume 308, June 19, 1978, entitled "Liposomes and their Uses in Biology and Medicine," edited by Demetrios Papahadjopoulos and in Liposomes in Immunobiology, edited by Baldwin H. Thom and Howard R. Sisk and published by Elseveir, North Holland, 1980, which discussions are incorporated herein by reference. For example, liposomes can be effectively made with phosphatidyl choline, cardiolipin, sphingomyelin, phosphatidyl ethanol amine, phosphatidyl inosotol, phosphatidyl serine, cholesterol, stearyl amine, and phosphatidic acid. In essence, any of the conventionally known liposome-forming lipids can be utilized to make liposomes in the process of the present invention. The conditions under which the liposomes are to be formed are those conventionally employed in the art for the specific materials which have been selected. The only alteration which has been found to be necessary is the utilization of the lipophilic chelating agent described previously in this specification. Depending on the type of lipisomes being prepared (e.g., positively charged, negatively charged, or neutral), the lipid ratio used in forming the liposomes will be varied according to the established practice of the art. In a preferred embodiment of the invention, cholesterol is included in the lipisomes to provide bilayer stability in addition to the bilayer stability produced by the lipophilic chelating agent.

The amount of the lipophilic chelating agent which is to be employed may vary broadly and is not critical to the present invention. The minimum amount to be used is that at which the yield of encapsulated drug is increased over the yield in the absence of the chelating agent and can be determined by simple experimentation. The maximum practical amount can be determined experimentally and as well is that beyond which no increase in yield is obtained, although greater amounts can be used if desired. Typical quantities of the lipophilic chelating agent to be used range from 0.1 to 50 percent by weight, preferably 1 to 25 percent by weight, still more preferably 5 to 15 percent by weight of the total chelating agent and liposome-forming material mass.

The amount of drug which will actually be encapsulated in each individual liposome will vary from drug to drug and indeed from liposome to liposome. However, it has been found that through the use of the present process one can achieve substantially increased total yields of drug encapsulation. A typical example of measuring the amount of drug incorporated is described in the following Examples.

The conditions under which the actual liposome encapsulation occurs will vary depending upon the specific lipids being utilized and are conditions which are well known in the art. Such techniques will typically involve introducing the lipophilic chelating agent and the drug into a solution containing the lipids which will form the liposome. The liposomes which can be formed may be positive, negative or neutral depending upon the lipids which are selected. In general, the drug, lipophilic chelating agent and lipids are stirred in the solution to form a dispersion, with the encapsulated product then being recovered by suitable techniques. Dispersion is generally undertaken by employing a magnetic stirrer or other stirring device for approximately 20 minutes to form a dispersion and/or by sonicating the solution. The sonicating process typically forms smaller liposomes while stirring without sonicating produces larger liposomes. The encapsulated product is isolated by removing free drug from the encapsulated material. Since the encapsulated material is not dialyziable, free drug is typically removed by dialysis using a membrane having pores of a size sufficient to retain the lipisomes but large enough to pass the free drug. After dialysis, the dialyzed material will consist of lipisomes and dialysis buffer, and the lipisomes can be readily recovered.

The weight of lipids used in forming the lipisomes will vary depending on the exact conditions used to form the lipisomes, and will typically range from about 2 times to about 6 times the weight of the active material.

A principal significance of the present invention lies in the fact that a substantially higher degree of encapsulation of therapeutic agents can be achieved in liposomes when a lipophilic chelating agent utilized. The hydrocarbon chain or other lipophilic portion anchors the molecule within the liposome bilayer while the polar DTPA (or any other chelating agent) group, being hydrophilic, is present on the liposome surface. The whole complex provides substantial spacing and stability to the lipid bilayers, thereby increasing the amount of drug being entrapped in the liposomes, dependent on their physicochemical properties. As evident from the present studies, for example, daunorubicin is encapsulated more than doxorubicin because the former is more lipophilic. In the same manner it would be reasonable to expect that other lipophilic drugs will be encapsulated in liposomes with a better efficiency than would less lipophilic drugs.

This invention is particularly useful in encapsulating antitumor drugs. These antitumor drugs can be classified as follows: 1) plant alkaloids e.g., vincristine, vinblastine, vindisine, VP-16; 2) antibiotics, e.g., Actinomycin D, doxorubicin, daunobicin, mitomycin C, bleomycin, and mitoxantrone; 3) antimetabolites, e.g., methotrexate, cytosine arabinoside, 6-mercaptopurine, and 5-azacytidine; 4) alkylating agents, e.g., melphalan, BCNU, CCNU, MeCCNU, and cis-platinum. One novelty of the present invention is that since the chelating agent, such as DTPA is present on the liposome surface, any metal-bearing, water-soluble drug, such as cis-platinum or CBDCA, can be captured in the liposome structure in a much more efficient way.

The invention will now be illustrated and explained by specific embodiments shown in drawings and examples, which are described below.

## Example 1

### Preparation of Octadecylamine-DTPA

The synthesis involves a reaction of the anhydride of diethylenetriaminepentaacetic acid (DTPA) in chloroform with octadecylamine (stearylamine). The DTPA anhydride was obtained commercially. Stearylamine was also obtained commercially at 98% purity and was used without further purification. About 2-3 gm of stearylamine was dissolved in 200 ml of anhydrous chloroform in a round-bottom flask. A 1.1- to 1.5-molar excess of DTPA anhydride was added to this mixture and was refluxed with stirring for 1-2 days. The reaction product was insoluble in chloroform and was collected by filtration. Formation of a reaction product insoluble in chloroform was indicative of amide bond formation. The reaction product was treated with boiling water to hydrolize the remaining anhydride linkage as well and purified by recrystallization from hot ethanol. The infra-red spectrum of this product is characterized by a strong band at 1640-1670 $cm^{-1}$ due to the amide linkage, the strong CH band due to saturated hydrocarbon chain, and the strong carbonyl stretching band. The purified stearylamine-DTPA reaction product was stored in a freezer for further studies.

## Example 2

### Preparation of Encapsulated Drugs

For the preparation of liposomes, 6 mg of doxorubicin or daunorubicin was added to 9 mg of cardiolipin solution and evaporated to dryness under $N_2$. To this dried mixture were then added 22.5 mg of phosphatidyl choline, 7.5 mg cholesterol and 3 mg of stearylamine-DTPA. Again, the mixture was evaporated to dryness under $N_2$.

The dried lipids and doxorubicin or daunorubicin were resuspended in 6 ml of 0.01 M phosphate buffer with 0.85% NaCl, pH 7.4 (PBS). After one-half-hour swelling time, the liposomes were stirred for 15 minutes, followed by sonication (Heat System, Model W-220F) in a fixed temperature bath at 37°C for 90 minutes. The non-entrapped doxorubicin or daunorubicin was separated from liposome-encapsulated drug by extensive dialysis against 0.001 M phosphate buffer with 0.85% NaCl, pH 7.4 at 4°C over a period of 20 hours with at least two changes of buffer solutions (250 ml/ml liposomes). The percentage of entrapment of drugs in these liposomes was determined by fluorescence after the completion of dialysis; it was found that doxorubicin was entrapped at an efficiency of 65-75% and daunorubicin was entrapped at an efficiency of 75-85%. The difference in encapsulation of these two drugs relates to their lipophilicity; daunorubicin is more lipophilic than doxorubicin. The liposomes were freshly prepared each day and were diluted with 0.01 M phosphate buffer (PBS) so as to administer doses of doxorubicin or daunorubicin equivalent to doses of free drugs for pharmacological, toxicological and therapeutic studies.

## Example 3

### Stability of Daunorubicin in Liposomes

The retention of daunorubicin in liposomes in the presence of buffer or plasma was followed at 4° and 37°C by a dialysis method. Table 1 shows that by 4 hrs the amount of drug released at 4°C in 0.01 M PBS was 3.6% which was raised to 11.4% by 24 hrs. The amount of drug released at 37°C was 18% at 4 hours. The same amount of drug was released if the liposomes were mixed with 50% plasma. Table 1 also shows that the release of drug from liposomes is temperature dependent.

TABLE 1

STABILITY OF DAUNORUBICIN ENTRAPPED IN LIPOSOMES IN BUFFER AND PLASMA

| Time of Analysis for drug release from liposomes (hr) | 0.01 M PBS at 4°C | 0.01 M PBS at 37° | Plasma 37° |
|---|---|---|---|
| 1 | 1.7 | 3.4 | 3.9 |
| 2 | 2.2 | 7.4 | 6.1 |
| 3 | 3.2 | 9.7 | 8.1 |
| 4 | 3.6 | 11.6 | 11.6 |
| 24 | 11.4 | -- | -- |

Example 4

Pharmacological Studies

Male $CDF_1$ mice weighing 20-25 g were used to evaluate the influence of liposome encapsulation on plasma levels and physiological disposition of daunorubicin and doxorubicin. Free or encapsulated daunorubicin or doxorubicin was administered intravenously via a lateral tail vein at a dose of 6 mg/kg at 2% body weight (0.02 ml/gm). At 5, 15, 30, 60, 120, 240, and 480 minutes and at 24 hours, mice in each group were bled from the orbital sinus. Blood was collected in heparinized tubes and centrifuged at 1500 rpm for 10 minutes. The plasma layer was separated and frozen. Mice were then killed by cervical dislocation, and the liver, kidney, heart, spleen, small intestine and lungs were excised, rinsed in normal saline, and stored at -20°C until assayed.

Plasma and tissues were analyzed for drug equivalents by the method of Formelli et al. (Cancer Chemotherapy and Pharmacology, 5, 129-144 (1981)) as modified in the inventor's laboratories. Plasma (0.25 ml) was diluted to 1 ml with distilled water, followed by addition of 0.2 ml of $AgNO_3$ (33% wt/vol). Tissues were homogenized in 1 ml of water in a polytron homogenizer. To each tube was then added 0.2 ml of $AgNO_3$. The tubes were vortexed vigorously followed by addition of 3 ml of n-butanol saturated with water. Each tube was vortexed for 1 minute and then was centrifuged at 5,000 rpm for 10 minutes. The organic layer was removed followed by further extraction of the residue with 2 ml of n-butanol. The tubes were vortexed for 30 seconds and then centrifuged for 10 minutes again at 5,000 rpm. The second organic layer was removed and pooled with the first extract. The butanol extract was read in a spectrofluorometer at 470 nm excitation and 585 nm emission. Control plasma and tissues obtained from mice treated with blank liposomes were treated the same way and read in the fluorometer to correct for any endogenous fluorescence. Blank liposomes contained DTPA-stearylamine but no drug. Freshly prepared standard samples of daunorubicin or doxorubicin in n-butanol were used each day to calculate the concentration of drugs in the plasma and tissues.

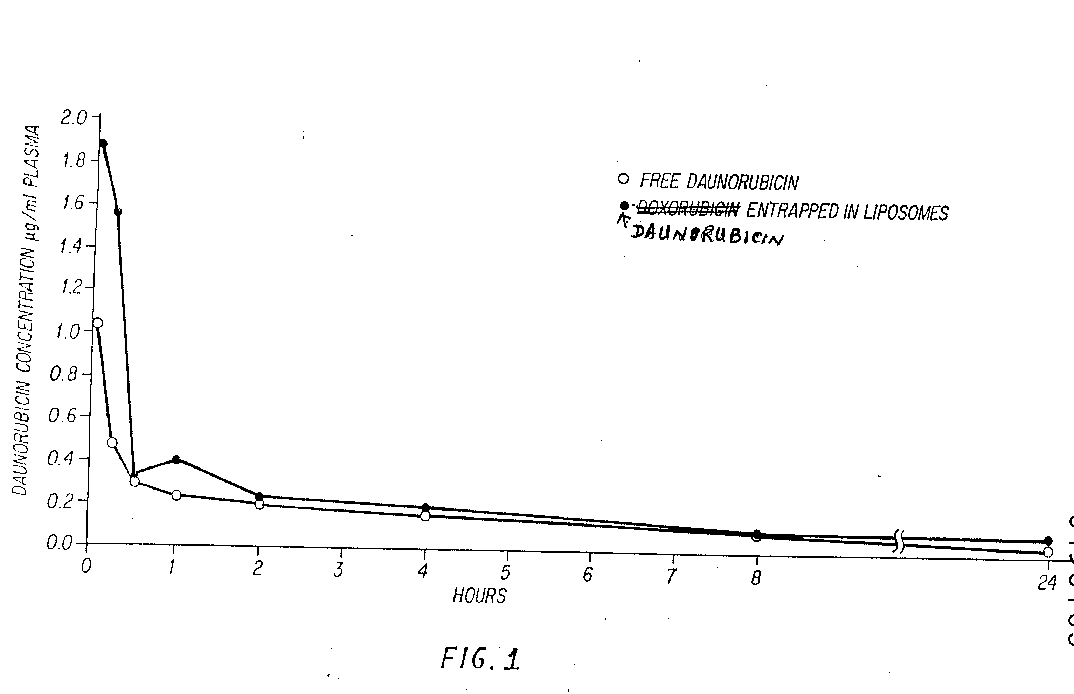

The respective plasma levels in mice following a dose of daunorubicin of 6 mg/kg as free drug or entrapped in liposomes are shown in FIGURE 1. The results are the average of the concentration of four animals per time. The peak plasma levels of daunorubicin-equivalent following free drug administration was 1 μg/ml, whereas the peak plasma concentration with liposomal daunorubicin was 1.9 μg/ml. By 30 minutes the levels of drug equivalents were similar in plasma either with free drug or with drug entrapped in liposomes. This situation lasted during the entire 24-hour time of observation.

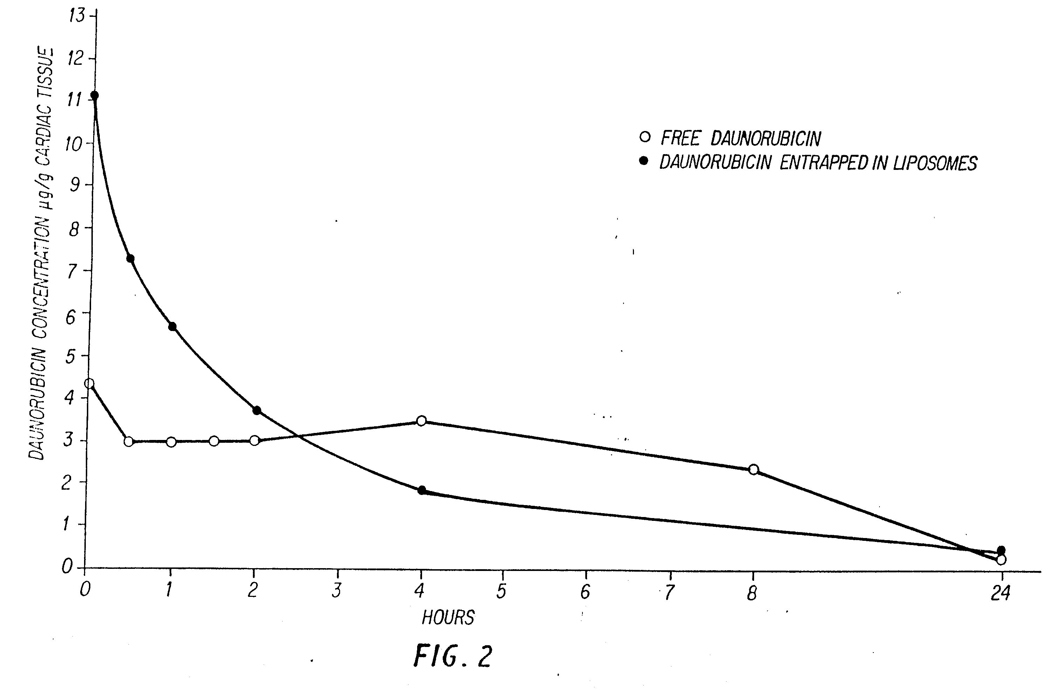

FIGURE 2 shows the cardiac uptake of free daunorubicin or daunorubicin entrapped in liposomes in $CDF_1$ mice. The peak drug concentration in heart occurred at 10 minutes both with free drug or drug entrapped in liposomes, the values being 11.2 and 4.4 μg/gm wet weight, respectively. The drug levels observed with liposomal daunorubicin in heart were half of those observed with free drug up to 1 hour. At 4 hours there was slightly more daunorubicin in cardiac tissue with liposomal drug than free drug. However, by 8 hrs and 24 hrs, the drug values in cardiac tissue were more or less similar with liposomal daunorubicin or free daunorubicin. This indicates that the cardiac uptake of liposomal daunorubicin is preferentially retarded at early time points compared to free drug. Such early times are more important than later times for controlling toxicity of the higher levels of drug present immediately after inoculation.

Table 2 presents the pharmacologic disposition of free and liposome-entrapped daunorubicin following i.v. administration of 6 mg/kg drug to $CDF_1$ mice. The drug

levels in liver and spleen were 2-3 fold higher with liposomal daunorubicin than with free drug during 5 minutes to 24 hrs. The levels of drug were equivalent in the lungs following administration of free and liposome-entrapped daunorubicin. On the contrary, the drug levels in the kidneys were at least two fold less with liposomal daunorubicin than free daunorubicin. The values of drug equivalents in the small intestine showed a modest but definite decrease in concentration of liposomal daunorubicin compared to free drug.

TABLE 2

PHARMACOLOGIC DISPOSITION OF FREE AND LIPOSOME-ENTRAPPED DAUNORUBICIN FOLLOWING I.V. ADMINISTRATION OF 6 mg/kg TO $CDF_1$ MICE

μg/gm Tissue

| Time | Liver | | Spleen | | Lungs | | Kidney | | Intestine | |
|---|---|---|---|---|---|---|---|---|---|---|
| | Free | Liposome | Free | Liposome | Free | Liposome | Free | Liposome | Free | Liposome |
| 5 min. | 29.6 | 52.5 | 8.8 | 29.8 | 18.9 | 17.6 | 111.0 | 38.6 | 13.8 | 8.6 |
| 15 min. | 25.4 | 36.5 | 15.2 | 80.3 | 16.4 | 17.3 | 35.6 | 28.5 | 17.9 | 8.4 |
| 30 min. | 20.6 | 43.3 | 10.6 | 41.5 | 14.1 | 11.8 | 89.6 | 29.2 | 3.7 | 7.2 |
| 1 hr. | 15.2 | 40.9 | 10.3 | 32.2 | 10.8 | 9.8 | 63.2 | 30.4 | 7.8 | 6.1 |
| 2 hr. | 11.2 | 33.1 | 13.2 | 17.0 | 8.0 | 8.2 | 54.3 | 23.8 | 6.9 | 6.1 |
| 4 hr. | 7.6 | 23.4 | 12.4 | 49.7 | 6.5 | 6.2 | 40.7 | 14.2 | 4.8 | 5.7 |
| 8 hr. | 6.0 | 14.4 | 12.5 | 46.5 | 3.8 | 4.1 | 28.9 | 10.0 | 4.3 | 3.7 |
| 24 hr. | 1.0 | 2.4 | 5.2 | 15.0 | 1.0 | 1.3 | 8.1 | 2.6 | 1.0 | 1.0 |

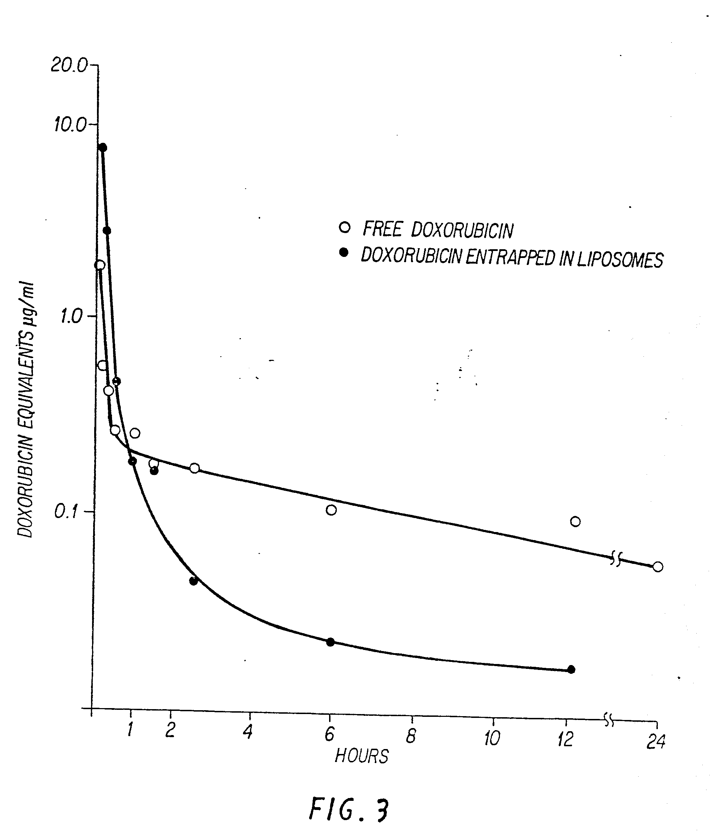

FIGURE 3 presents the plasma levels of doxorubicin in mice, following a dose of 6 mg/kg as free drug or entrapped in liposomes. The peak plasma concentration with free drug was 2.4 μg/ml, whereas with liposomal doxorubicin the peak plasma concentration was 9.2 μg/ml, about 4-fold higher. The plasma concentration with liposomal doxorubicin up to 30 minutes after inoculation was significantly higher than free drug, and thereafter the values were signifiantly less than free drug up to 24 hrs.

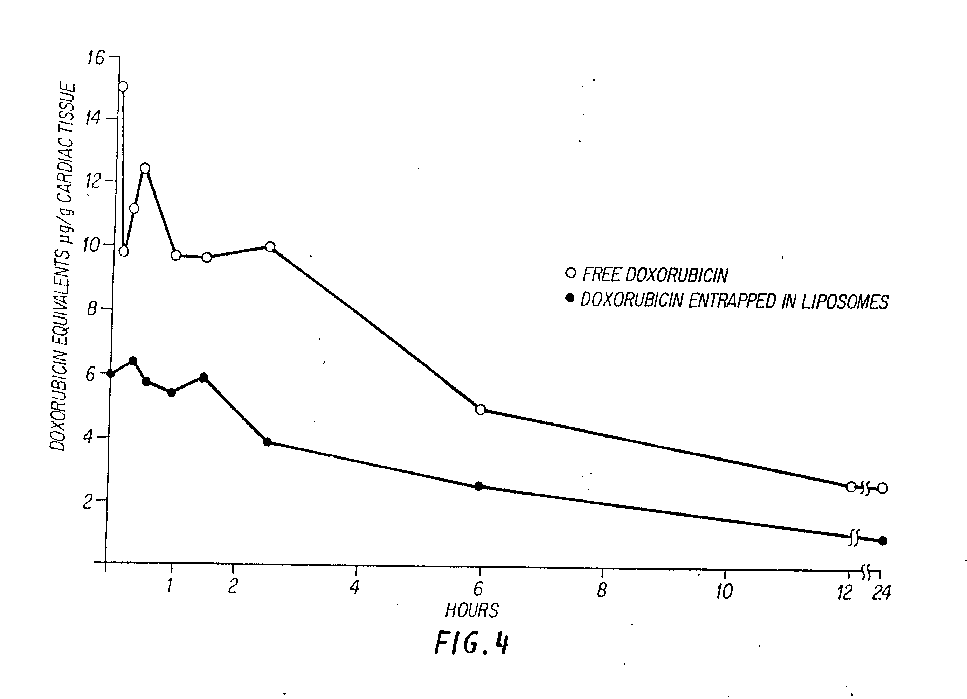

FIGURE 4 presents the cardiac drug levels following administration of free and liposomal doxorubicin. The peak cardiac concentration with free doxorubicin was achieved at 10 minutes, the values being 15.2 μg/gm of tissue. However, with liposomal doxorubicin, the peak cardiac concentration was 6.0 μg/gm, obtained also at 10 minutes following drug administration. From 10 minutes to 24 hours, the values of drug equivalent in cardiac tissue with liposomal doxorubicin were at least 2- to 4-fold less than free drug. It is evident that uptake of doxorubicin in cardiac tissue is specifically restricted when administered entrapped in liposomes.

Table 3 presents the pharamcologic disposition of liposome-entrapped doxorubicin and free doxorubicin following administration of 6 mg/kg dose i.v. to $CDF_1$ mice. The levels of drug in the liver were quantitatively similar up to 1/2 hour when administered as either free or entrapped in liposomes. However, after 1/2 hour, the drug levels were about two-fold higher with liposomal doxorubicin than free drug. The levels of drug equivalents in the spleen with doxorubicin entrapped in liposomes were increased 3- to 5-fold at all times of observation compared to free drug. In contrast, drug levels in the kidney were markedly reduced with liposomal delivery; with free doxorubicin the peak drug concentration was 41.0 μg/gm, whereas with liposomes the peak drug levels were 14.0 μg/gm at 15 minutes. The levels in the lungs were more or less equivalent with free doxorubicin or doxorubicin entrapped in liposomes at all periods of observation.

TABLE 3

PHARMACOLOGIC DISPOSITION OF FREE AND LIPOSOME-ENTRAPPED DOXORUBICIN FOLLOWING I.V. ADMINISTRATION OF 6 mg/kg TO $CDF_1$ MICE

µg/gm Tissue

| | Liver | | Spleen | | Lung | | Kidney | | Intestine | |
|---|---|---|---|---|---|---|---|---|---|---|
| | Free | Liposome | Free | Liposome | Free | Liposome | Free | Liposome | Free | Liposome |
| 5 min. | 44.5 | 40.4 | 12.6 | 39.0 | 11.3 | 18.3 | 47.0 | — | 3.8 | 5.4 |
| 15 min. | 37.3 | 48.2 | 9.7 | 40.7 | 8.3 | 12.3 | 41.0 | 14.0 | — | 8.0 |
| 30 min. | 50 | 43.8 | 12.1 | 33.6 | 10.1 | 9.9 | 41.5 | 11.4 | 8.4 | 7.3 |
| 1 hr. | 23.2 | 45.4 | 10.0 | 39.5 | 6.6 | 7.9 | 24.4 | 14.1 | — | 7.9 |
| 1.5 hr. | 24.9 | 40.5 | 10.9 | 62.0 | 6.5 | 14.9 | 32.9 | — | — | 4.9 |
| 2.5 hr. | 22.7 | 32.3 | 13.1 | 24.0 | 6.8 | 6.2 | 29.5 | 5.0 | 5.0 | 4.7 |
| 6 hr. | 11.4 | 16.2 | 13.9 | 28.0 | 3.5 | 5.1 | 16.6 | 2.7 | 2.7 | 2.5 |
| 24 hr. | 3.6 | 9.1 | 11.7 | 17.8 | 3.0 | 2.5 | 5.4 | 0.4 | 1.6 | 1.8 |

## Example 5

### Antitumor Studies

To investigate the therapeutic effectiveness of doxorubicin and daunorubicin entrapped in liposomes, antitumor studies were performed against P388 leukemia in $CDF_1$ mice. Groups of ten mice were implanted intraperitoneally with $1x10^6$ cells of P388 tumor, and drug treatment was initiated on day one intraperitoneally or intravenously after tumor cell inoculation. Antitumor efficacy was expressed in leukemic animals by the increase in median survival time, in the dose-range used, compared to controls. Dead animals were autopsied and toxicity was established on the basis of macroscopic necropsy findings.

Table 4 presents the antitumor response of either free doxorubicin or doxorubicin entrapped in liposomes at a dose of 15 and 20 mg/kg. With free doxorubicin at a dose of 15 mg/kg i.p., the median T/c was 130%, and the same T/c was observed with liposomal drug. At a dose of 20 mg/kg i.p. of free doxorubicin, a T/c of 100 was achieved, whereas the same dose of drug entrapped in liposomes gave a T/c of 110. These studies demonstrate that encapsulation of doxorubicin in liposomes does not compromise the therapeutic activity of the drug.

TABLE 4

ANTITUMOR RESPONSE OF FREE DOXORUBICIN OR DOXORUBICIN ENTRAPPED IN LIPOSOMES AGAINST P388 LEUKEMIA

| Dose | Route | Free Doxorubicin *T/c (%) | Doxorubicin-Liposomes T/c (%) |
|---|---|---|---|
| 15 mg/kg | i.p | 130 | 130 |
| 20 mg/kg | i.p. | 100 | 110 |

Mice were implanted i.p. $1x10^6$ cells of murine P388 leukemia and 24 hours later free doxorubicin or doxorubicin entrapped in liposomes was administered i.p.

Table 5 presents the antitumor response of daunorubicin as a free drug or entrapped in liposomes against P388 leukemia in mice. At a dose of 2 or 4 mg/kg i.p. with free daunorubicin or daunorubicin entrapped in liposomes, equivalent antitumor activity was achieved. However, at a dose of 6 mg/kg i.p. with daunorubicin a T/c of 109 was achieved whereas with liposomal drug a T/c of 127 was obtained. Intravenous administration of free daunorubicin and daunorubicin entrapped in liposomes at doses of 15 and 20 mg/kg to mice bearing P388 leukemia i.p. exhibited equivalent antitumor activity. These studies further demonstrate that liposomal entrappment of daunorubicin does not compromise with therapeutic efficacy of these important antitumor drugs.

TABLE 5

ANTITUMOR EVALUATION OF DAUNORUBICIN FREE AND ENTRAPPED IN LIPOSOMES AGAINST P338 LEUKEMIA

| Dose | Route | Free Daunorubicin T/c (%)* | Daunorubicin-Liposomes T/c (%) |
|---|---|---|---|
| 2 mg/kg | i.p. | 145.5 | 145.5 |
| 4 mg/kg | i.p. | 136.4 | 145.5 |
| 6 mg/kg | i.p. | 109.1 | 127.3 |
| 15 mg/kg | l.v | 145.5 | 136.4 |
| 20 mg/kg | i.v. | 154.5 | 145.5 |

Mice were implanted i.p. $1x10^6$ cells of murine P388 leukemia and 24 hours later free liposome-entrapped daunorubicin was administered i.p. or i.v.

* $\frac{\text{Treated}}{\text{Control}}$ x 100

Example 6

Toxicity Evaluation of Doxorubicin Entrapped in Liposomes

For comparison of lethal toxicity of free doxorubicin and doxorubicin entrapped in liposomes, normal $CDF_1$ mice were administered a dose of 20 mg/kg intraperitonally. The mice were observed until day 80 post-drug administration. The control mice received blank liposomes representing the same concentration of the lipid used to entrap a dose of 20 mg/kg of doxorubicin. Mice in each group were weighed twice weekly and were observed daily.

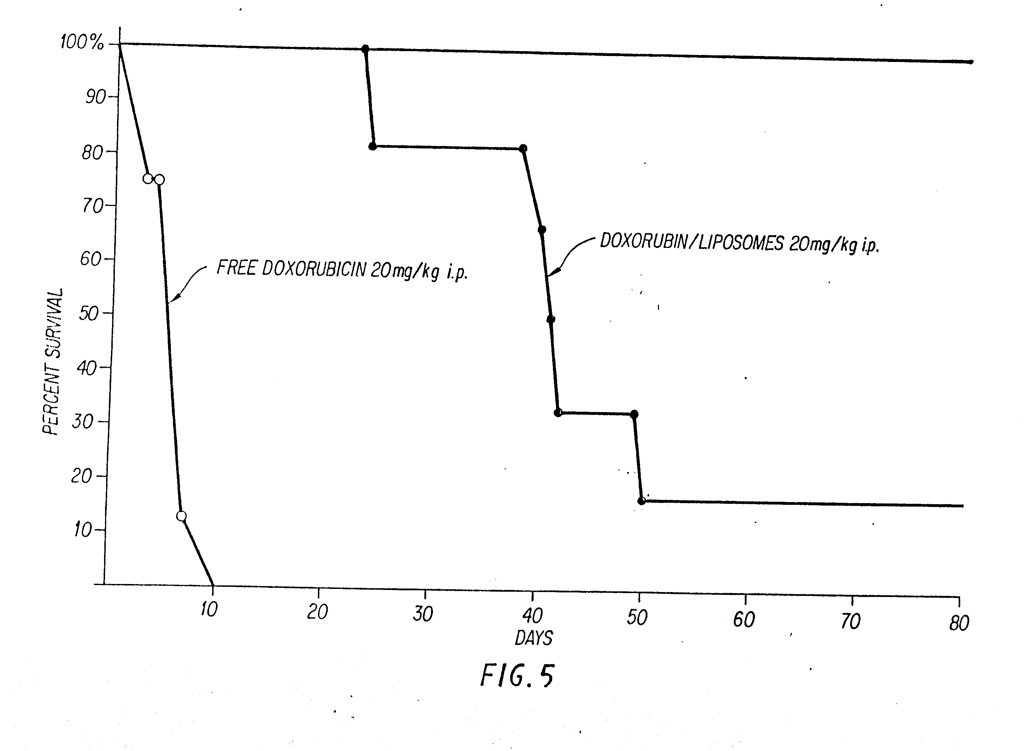

FIGURE 5 compares the lethal toxicity of free doxorubicin and doxorubicin entrapped in liposomes. Animals treated with free doxorubicin at a dose of 20 mg/kg exhibited 100% mortality by day 10. However, animals treated with doxorubicin entrapped in liposomes at a dose of 20 mg/kg demonstrated only 10% mortality by day 19 and no toxic deaths were observed until day 38. At day 80, 20% of the mice which were treated with liposomal encapsulated drug were still surviving. This shows that entrapment of doxorubicin in liposomes markedly reduces the acute toxicity of drug when compared to free doxorubicin. Furthermore, the blank liposomes are shown to be devoid of any toxicity when injected into mice without the drug.

The invention now being fully described, it will be apparent to one of ordinary skill in the art that many changes and modifications can be made thereto without departing from the spirit or scope of the invention as set forth herein.

Claims

1. A process for encapsulating a physiologically active substance in a liposome which comprises:

(a) forming a dispersion of a liposome-forming lipid, a physiologically active substance, and a compound of formula R-K, wherein R is a lipophilic portion of said compound which is capable of inserting into a liposomal lipid bilayer and K is a chelating portion of said compound, whereby liposomes are produced in said dispersion; and

(b) recovering the liposome-encapsulated physiologically active substance from said dispersion.

2. The process of Claim 1, wherein said dispersion is formed by stirring a mixture comprising water, the liposome-forming lipid, compound R-K, and the physiologically active substance.

3. The process of Claim 1, wherein said dispersion is sonicated prior to recovering the liposomes.

4. The process of Claim 1 wherein the physiologically active substance is doxorubicin, daunorubicin or cis-platinum.

5. The process of Claim 1, wherein said compound of formula R-K is a covalent compound formed by the condensation reaction of a polycarboxylic chelating agent and an alkyl or alkenyl amine or hydroxide.

6. In a process for producing a liposome-encapsulated physiologically active substance, an improvement which comprises incorporating a compound of formula R-K, wherein R is a lipophilic portion of said compound that is capable of inserting into a liposomal lipid bilayer and K is a chelating portion of said compound, into a mixture of liposome-forming lipid and physiologically active substance from which said liposomes are being prepared.

7. The liposome-encapsulated physiologically active substance produced by the process of Claim 1.

8. In a liposome-encapsulated drug composition, an improvement which comprises a lipophilic chelating agent entrapped in said composition.

9. The composition of Claim 8, wherein the lipophilic chelating agent is formed by a condensation reaction between diethylenetriaminepentaacetic acid or ethylenediaminetetracetic acid and an amine of the formula $RNH_2$, wherein R is an alkyl group containing from 12 to 22 carbon atoms or an alkenyl group containing from 12 to 22 carbon atoms and at least one double bond.

10. A liposome-encapsulated physiologically active substance, wherein said liposome comprises a bilayer having inserted therein an R portion of a compound of formula R-K wherein R is a lipophilic portion of said compound and K is a chelating portion of said compound.

11. The liposome-encapsulated physiologically active substance of Claim 10, wherein R comprises a hydrocarbyl portion comprising at least 6 carbon atoms.

12. The liposome-encapsulated physiologically active substance of Claim 11, wherein said hdyrocarbyl portion comprises at least 12 carbon atoms.

13. The liposome-encapsulated physiologically active substance of Claim 11, wherein R is an alkenyl group.

14. The liposome-encapsulated physiologically active substance of Claim 11, wherein R is an alkyl group.

15. The liposome-encapsulated physiologically active substance of Claim 14, wherein R is a dodecyl, tridecyl, tetradecyl, pentadecyl, hexadecyl, heptadecyl, octadecyl, nonadecyl, icosyl, henicosyl, or docosyl group.

16. The liposome-encapsulated physiologically active substance of Claim 10, where K chelates through at least one carboxylic acid group.

17. The liposome-encapsulated physiologically active substance of Claim 16, where K comprises an aminocarboxylic acid, hydroxycarboxylic acid, or polycarboxylic acid.

18. The liposome-encapsulated physiologically active substance of Claim 16, wherein K comprises a portion of formula $-N(K^1)CH_2CH_2N(K^2)(K^3)$ wherein $K^1$, $K^2$, and $K^3$ independently represent H or $-CH_2CO_2H$, with the proviso that no more than two of $K^1$, $K^2$, and $K^3$ are H.

19. The liposome-encapsulated physiologically active substance Claim 18, wherein R-K is a condensation product of diethylenetriaminepentaacetic acid or ethylenediaminetetraacetic acid and an amine formula $RNH_2$ or an alcohol of formula ROH.

20. The liposome-encapsulated physiologically active substance Claim 10, wherein said physiologically active substance is doxarubicen or daunorubicin.

○ FREE DAUNORUBICIN
● DOXORUBICIN ENTRAPPED IN LIPOSOMES
DAUNORUBICIN
DAUNORUBICIN CONCENTRATION μg/ml PLASMA
2.0
1.8
1.6
1.4
1.2
1.0
0.8
0.6
0.4
0.2
0.0
0
1
2
3
4
5
6
7
8
24
HOURS

FIG. 1

○ FREE DAUNORUBICIN
● DAUNORUBICIN ENTRAPPED IN LIPOSOMES
DAUNORUBICIN CONCENTRATION μg/g CARDIAC TISSUE
0
1
2
3
4
5
6
7
8
9
10
11
12
13
0
1
2
3
4
5
6
7
8
24
HOURS

FIG. 2

20.0
10.0
1.0
0.1
DOXORUBICIN EQUIVALENTS μg/ml
FREE DOXORUBICIN
DOXORUBICIN ENTRAPPED IN LIPOSOMES
1
2
4
6
8
10
12
24
HOURS


FIG. 3

0198765

○ FREE DOXORUBICIN
● DOXORUBICIN ENTRAPPED IN LIPOSOMES
DOXORUBICIN EQUIVALENTS µg/g CARDIAC TISSUE
16
14
12
10
8
6
4
2
1
2
4
6
8
10
12
24
HOURS

FIG. 4

100%
90
80
70
60
50
40
30
20
10
PERCENT SURVIVAL
FREE DOXORUBICIN 20mg/kg i.p.
DOXORUBIN/LIPOSOMES 20mg/kg i.p.
10
20
30
40
50
60
70
80
DAYS

FIG. 5